# EUROPEAN PATENT SPECIFICATION

(11) **EP 0 581 258 B1**
(45) Date of publication and mention of the grant of the patent: **11.12.1996**
(21) Application number: 93112036.4
(22) Date of filing: 28.07.1993
(51) Int. Cl.: A61F 13/15

(54) **Absorbent Product**
Absorbierendes Produkt
Produit absorbant

(30) Priority: 30.07.1992 JP 223347/92
(43) Date of publication of application: 02.02.1994
(73) Proprietor: SYTIK CORPORATION, Tokyo (JP)
(72) Inventor: Fukui, Hiroaki, Kawaguchi-shi , Saitama (JP)
(74) Representative: Gervasi, Gemma, Dr.

(56) References cited:
- EP-A- 0 479 442
- GB-A- 2 143 439
- US-A- 4 285 343
- US-A- 4 608 047
- US-A- 4 904 251
- PATENT ABSTRACTS OF JAPAN, unexamined applications, C section, vol. 15, no. 425, October 29, 1991, THE PATENT OFFICE JAPANESE GOVERNMENT page 57 C 879

## Description

### BACKGROUND OF THE INVENTION

The present invention relates to an absorbent product, such as a sanitary napkin, which is worn in contact with the skin of the wearer for the purpose of absorption of body liquids and, more particularly, to an absorbent product equipped with a wing for fixing to an underwear of the wearer.

There have been widely used such type of absorbent products for sanitary napkins provided with a pair of wings which are used to fixedly attach the napkin to an underwear, for the purpose of preventing displacement from the desired position while being worn.

Such type of the winged absorbent product is described in, for example, Japanese laid-open patent application No. Hei 3-176050. The well known product comprises a liquid-impermeable back sheet and a liquid-permeable top sheet, between which a layer of a absorbent material is accommodated, and a pair of wings formed by projecting part of the back sheet at both sides thereof in a desired form.

On the other hand, in order to prevent a so-called lateral leakage of the body liquid, that is, undesirable leakage of the body liquid out of the longitudinal side edges of the absorbent product when the excessive body liquid is supplied, it has been well known to provide a pair of side barrier members disposed so as to enclose the longitudinal edge portions on both sides of the absorbent product (see Japanese laid-open U.M. application No. Hei 3-88511). The side barrier members are provided by adhering liquid-impermeable sheets to both sides of the absorbent product over its entire length.

However, in the foregoing conventional winged absorbent product, it is necessary to adhere a band-shaped liquid-impermeable sheet prepared independently of the wings above the top sheet of the absorbent body, thereby causing the production process complicated. In addition, the cut out portion of the blank sheet material out of the barrier members cannot be used and wasted thereby to cause increase in the cost of the product.

### SUMMARY OF THE INVENTION

According to the present invention, there is provided an absorbent product comprising:
an absorbent body comprising a liquid-permeable top sheet a liquid-impermeable back sheet and an absorbent material accommodated between said top and back sheets; and
a pair of pieces comprising a liquid-impermeable sheet and disposed on the top sheet at both side edges of the absorbent body;
each of the pieces comprising a first portion disposed on the top sheet and extending along part of the both side edges of the absorbent body to form a side barrier and a second portion extending outwardly from the longitudinal side edges to form a wing, and being secured to the absorbent body at a connecting line extending along the boundary between the first and second portions.

In the absorbent product according to the present invention, the wings and the side barrier members are made of the same piece. Each of the portions protruding outwardly from the longitudinal side edges of the absorbent body functions as wings, and the portion lying inwardly of the longitudinal side edge of the absorbent body functions as the side barrier member. It should be noted that the side barrier members are formed at the restricted portions of the entire length of the absorbent body.

Therefore, the absorbent product of the present invention can be manufactured in a more simple production process as compared with the conventional absorbent product in which the wings and the side barrier members are separately provided. In addition, the barrier portion lies only at the portion which is necessary to prevent lateral leakage of the body liquid, and the waste of material can be eliminated.

In a preferred embodiment of the present invention, the side barrier members are provided so as to extend from the central portion of the absorbent body rearward. Thus, rearward leakage as well as the lateral leakage of the body liquid can be prevented.

The side barrier members may be provided with an elastic material attached to the edge portion thereof, so that its edge portion may be closely attached to the wearer's body. However, if the sheet constituting the side barrier members are made of a suitable stretchable material, then the both ends thereof can be joined to the absorbent body with this portion stretched to eliminate the attachment of the elasitic body. In any case, the wing members will stand upright, and perform a function of preventing the "lateral leakage".

Furthermore, according to the present invention, as compared with the conventional product wherein each wing and side barrier are formed in separate parts, the production process is greatly simplified and the productivity is improved, thereby reducing the production costs. In addition, the material to be wasted can be greatly decreased, which also lands itself to the reduction of cost.

Other features and advantages of the present invention will become readily apparent from the following detailed description with reference to the accompanying drawings.

### BRIEF DESCRIPTION OF THE DRAWINGS

FIGURE 1 illustrates in plan an absorbent product embodying the present invention as viewed from the top sheet;
FIGURE 2 is a cross-sectional view taken along the line II-II in FIGURE 1;
FIGURE 3 is a plan view showing another absorbent product embodying the present invention as viewed from the top sheet;
FIGURE 4 is a plan view, as viewed from the top sheet, of another absorbent product embodying the present invention;
FIGURE 5 is an enlarged cross-sectional view taken along the line V-V in FIGURE 4;
FIGURE 6 illustrates part of a continuous sheet from which pieces for the wings of the product of FIGURE 1 are cut out along the cutting lines; and
FIGURE 7 illustrates part of a continuous sheet from which pieces for the wings of the product of FIGURE 4 are cut out along the cutting lines.

### DESCRIPTION OF THE PREFERRED EMBODIMENTS

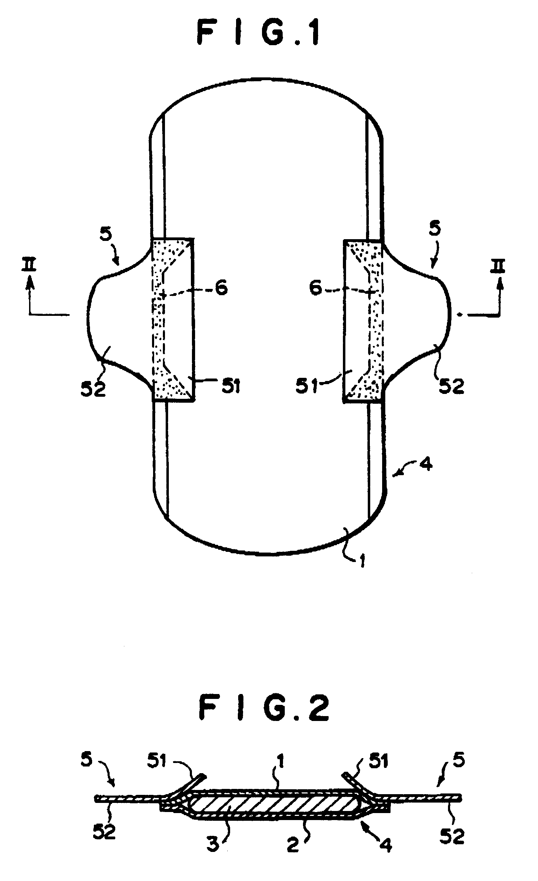

In FIGURES 1 and 2, an absorbent product embodying the present invention, which is formed for a sanitary napkin, comprises an absorbent body 4 joining a top sheet 1 of a liquid-permeable sheet and a back sheet 2 of a liquid-impermeable sheet, both having substantially same shape. The top and back sheets are connected together along the peripheral edges thereof, between which an absorbent body 3 is accommodated. A pair of pieces 5 are disposed above the top sheet 1 and attached to the body 4 at the connecting area or zone 6 by means of adhering or welding.

Each piece 5 comprises a first portion 51 laid on a top sheet 1 of the absorbent body 4 and a second portion 52 extending outwardly from the longitudinal side edge of the absorbent body 4, and secured to the absorbent body 4 at the connecting zone 6. Preferably, the connecting zone 6 extends along the longitudinal side edge of the absorbent body 4 at the boundary portion between the first and second portions 51 and 52, with leaving a portion which is not connected to the absorbent body at the free end of the first portion 51.

Therefore, the first portion 51 of the piece 5 forms a pocket in conjunction with the upper surface of the top sheet 1 and functions as a barrier for stopping the liquid traveling from the center of the absorbent body 4 toward the longitudinal side edge thereof.

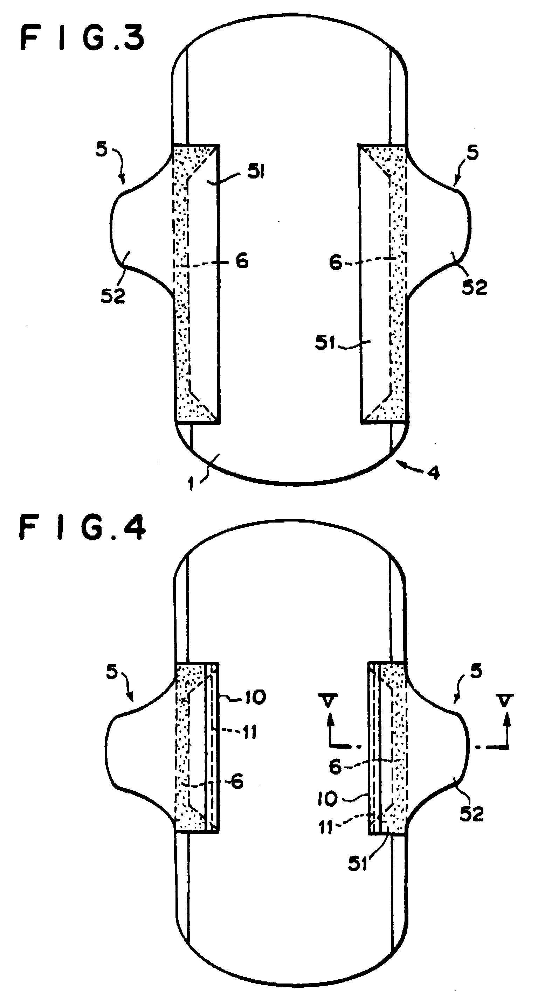

In the embodiment illustrated in FIGURE 1, the first portion 51 of the piece 5 has a length approximately equal to one-fourth to three-fourths of the entire length of the absorbent body 4. That is, the first portion 51 can cover about one half to three-fouths of the entire length of the absorbent body 4. For the product specifically intended to prevent the rearward leakage of the body liquid, the first portion 51 of the piece 5 has a length of about two thirds of the length of the absorbent body 4 so as to reach the rear end of the absorbent body 4, as shown in FIGURE 3.

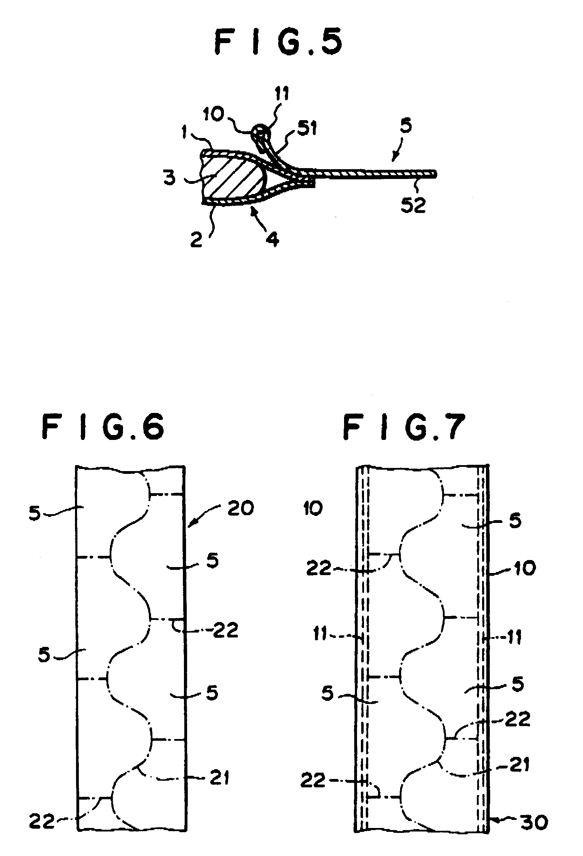

In the arrangements illustrated in FIGURES 1 and 3, part of the first portion 51 of the piece 5 may be folded back along the inner edge portion as shown in FIGURES 4 and 5, thereby to form a folded portion 10 within which an elastic member such as rubber thread 11 is disposed. The rubber thread 11 is securely fitted at both ends thereof to the piece 5 with being slightly stretched. If disired, the entire lenght of the elastic member may be bonded under a slightly streched condition to the folded portion 10 of the piece 5.

The rubber thread 11 causes the first portion 51 of the piece 5 standing up above the upper surface of the top sheet 1 when the absorbent body 4 is curved along the configuration of the wearer, thereby to achieve an extremely excellent effect of preventing the lateral leakage of body liquid.

In the absorbent product of the present invention, the pieces 5 are disposed on the liquid-permeable top sheet 1 of the absorbent body 4, that is, the side which faces the wearer's body. And, the second portion 52 of each piece 5 extends from the edge of the absorbent body 4, and serves as a wing. Upon wearing, as in the normal winged absorbent product, the second portions 52 or wings may be folded back over part of the underwear and fixed to the underwear at the outside or connected to each other.

In order to facilitate such fitting, a layer of a suitable pressure-sensitive adhesive (not shown) is provided at each top end portion of the second portion 52 and a peel-off sheet (not shown) temporarily attached thereon.

The piece 5 having the shape as shown in FIGURE 1 or 3 has a sufficient effect of preventing lateral leakage, even when it has a small area. This means that the amount of material to be used can be remarkably reduced, and also eliminate the portions of the material, when the pieces 5 are cut out from the continuous sheet material.

FIGURE 6 illustrates cutting lines depicted by 21 and 22 for cutting out the pieces 5 from a continuous sheet material 20 for use in the absorbent product shown in FIGURE 1. As can be seen from this figure, the cutting line 21 forms a substantially sine curve at the central portion of the sheet material, and each of the cutting lines 22 extends perpendicularly to the longitudinal direction and connects each peak of the cutting line 21 to both side edges. Therefore, it is possible that pairs of the pieces 5 is obtained at both sides of the sheet material 20 with no substantial portion to be wasted.

The pieces 5 having a structure as shown in FIGURES 4 and 5 may be formed by using a continuous sheet material 30 shown in FIGURE 7. The sheet material 30 comprises a pair of folded portions 10 at both side edges to form narrow passages through which rubber threads 11 are disposed under stretched condition. By cutting this sheet material 30 along the cutting lines 21 and 22 as described with reference to FIGURE 6, pieces 5 with rubber thread can be obtained. With the respective piece 5, the rubber thread 11 may be secured at both ends thereof in advance to the cutting by means of heat seal.

As has been described above, it will be understood that various modifications and variations can be effected without departing from the scope of the present invention.

## Claims

1. An absorbent product comprising:
an absorbent body (4) comprising a liquid-permeable top sheet (1), a liquid-impermeable back sheet (2) and an absorbent material (3) accommodated between said top and back sheets; and
a pair of pieces (5) comprising a liquid-impermeable sheet and disposed on the top sheet at both side edges of the absorbent body;
each of the pieces (5) comprising a first portion (51) disposed on the top sheet and extending along part of the both side edges of the absorbent body to form a side barrier and a second portion (52) extending outwardly from the longitudinal side edges to form a wing, and being secured to the absorbent body at a connecting zone (6) extending along the boundary between the first and second portions.

2. The absorbent product according to claim 1 wherein the second portion (52) of the piece (5) has a length of about one half of that of the absorbent body (4) and disposed at the substantially central portion of the longitudinal side edges of the absorbent body.

3. The absorbent product according to claim 1 wherein the first portion (51) of the piece (5) has a length of about two third of that of the absorbent body (4), and one end of the first portion reaches near the rear end of the absorbent body.

4. The absorbent product according to any of the claims 1 to 3 wherein a elastic material (11) is attached to the inner edge of the first portion (51) of the piece (5) under stretch condition.

5. A sanitary napkin formed of the absorbent product according to any one of claims 1 to 4.

6. A method of making the sanitary napkin according to claim 5 wherein the piece (6) is cut out from a continuous sheet material (20, 30) along a first cutting line (21) and a second cutting lines (22), the first cutting line extending along the longitudinal direction at the central portion of the sheet material to form a generally sine curve, and each of the second cutting lines extending perpendicularly to the side edges of the sheet material to connect the respective peak of the first cutting line to the side edge of the sheet material.

## Patentansprüche

1. Absorbierendes Produkt mit:
einem absorbierenden Körper (4), der eine flüssigkeitsdurchlässige obere Lage (1), eine flüssigkeitsundurchlässige untere Lage (2) und ein absorbierendes Material (3) aufweist, das zwischen der oberen und der unteren Lage abgeordnet ist; und
einem Teilepaar (5), das eine flüssigkeitsundurchlässige Lage aufweist und auf der oberen Lage an beiden Seitenrändern des absorbierenden Körpers angeordnet ist;
wobei jedes der Teile (5) einen ersten Abschnitt (51), der auf dar oberen Lage angeordnet ist und sich entlang eines Teils der beiden Seitenränder des absorbierenden Körpers erstreckt, um einen seitlichen Schutz zu bilden, und einen zweiten Abschnitt (52) aufweist, der sich von den Längsseitenrändern nach außen hin erstreckt, um einen Flügel zu bilden, und dieses an dem absorbierenden Körper an einem Verbindungsbereich (6) befestigt ist, der sich entlang der Grenze zwischen dem ersten und dem zweiten Abschnitt erstreckt.

2. Absorbierendes Produkt nach Anspruch 1, wobei der zweite Abschnitt (52) des Teils (5) eine Länge von etwa der Hälfte derjenigen des absorbierenden Körpers (4) besitzt und im wesentlichen an dem Mittenabschnitt der Längsseitenränder des absorbierenden Körpers angeordnet ist.

3. Absorbierendes Produkt nach Anspruch 1, wobei der erste Abschnitt (51) des Teils (5) eine Länge von etwa zwei Dritteln derjenigen des absorbierenden Körpers (4) besitzt und ein Ende des ersten Abschnitts fast bis an das rückwärtige Ende des absorbierenden Körpers reicht.

4. Absorbierendes Produkt nach einem der Ansprüche 1 bis 3, wobei ein elastisches Material (11) an dem Innenrand des ersten Abschnitts (51) des Teils (5) in gedehntem Zustand angeordnet ist.

5. Damenbinde, die aus dem absorbierenden Produkt gemäß einem der Ansprüche 1 bis 4 ausgebildet ist.

6. Verfahren zum Herstellen der Damenbinde gemäß Anspruch 5, wobei das Teil (5) aus einem fortlaufenden Lagenmaterial (20, 30) entlang einer ersten Schnittlinie (21) und zweiter Schnittlinien (22) ausgeschnitten wird, wobei sich die erste Schnittlinie entlang der Längsrichtung an dem Mittenabschnitt des Lagenmaterials erstreckt, um im wesentlichen eine Sinuskurve zu bilden, und jede der zweiten Schnittlinien sich senkrecht zu den Seitenrändern des Lagenmaterials erstreckt, um die jeweiligen Scheitelpunkte der ersten Schnittlinie mit dem Seitenrand des Lagenmaterials zu verbinden.

## Revendications

1. Produit absorbant comprenant :
un corps absorbant (4) comprenant une feuille supérieure (1) perméable aux liquides, une feuille de support (2) imperméable aux liquides et un matériau absorbant (3) situé entre ces feuilles supérieure et de support; et
deux morceaux (5) comprenant une feuille imperméable aux liquides et disposés sur la feuille supérieure au niveau des deux bords latéraux du corps absorbant;
chacun des morceaux (5) comprenant une première partie (51) disposée sur la feuille supérieure et se prolongeant le long d'une partie des deux bords latéraux du corps absorbant pour former une barrière latérale et une seconde partie (52) dirigée vers l'extérieur à partir des bords latéraux longitudinaux de façon à former un pan, et étant fixé au corps absorbant au niveau d'une ligne de raccordement (6) se prolongeant le long de la frontière entre les première et seconde parties.

2. Produit absorbant suivant la revendication 1, dans lequel la seconde partie (52) du morceau (5) a une longueur qui représente environ la moitié de celle du corps absorbant (4) et est disposée pratiquement dans la partie centrale des bords latéraux longitudinaux du corps absorbant.

3. Produit absorbant suivant la revendication 1, dans lequel la première partie (51) du morceau (5) a une longueur qui représente environ les deux tiers de celle du corps absorbant 4, et une extrémité de la première partie atteint le voisinage de l'extrémité arrière du corps absorbant.

4. Produit absorbant suivant l'une quelconque des revendications 1 à 3, dans lequel un matériau élastique (11) est attaché au bord intérieur de la première partie (51) du morceau (5) dans un état étiré.

5. Couche pour bébé formée du produit absorbant suivant l'une quelconque des revendications 1 à 4.

6. Procédé pour la fabrication de la couche suivant la revendication 5, dans lequel le morceau (5) est découpé dans une matière en feuille continue (20, 30), le long d'une première ligne de découpe (21) et de secondes lignes de découpe (22), la première ligne de découpe se prolongeant selon la direction longitudinale dans la partie centrale de la matière en feuille de façon à former une courbe à peu près sinusoïdale, et chacune des secondes lignes de coupe se prolongeant perpendiculairement aux bords latéraux de la matière en feuille pour raccorder le pic respectif de la première ligne de découpe au bord latéral de la matière en feuille.
